# EUROPÄISCHE PATENTSCHRIFT

(11) **EP 1 152 703 B1**
(45) Veröffentlichungstag und Bekanntmachung des Hinweises auf die Patenterteilung: **26.05.2004**
(21) Anmeldenummer: 00916839.4
(22) Anmeldetag: 16.02.2000
(51) Int. Cl.: A61B 17/68

(54) **IMPLANTAT ZUR FESTLEGUNG EINER KNOCHENPLATTE**
IMPLANT FOR FIXING A BONE PLATE
IMPLANT POUR FIXER UNE PLAQUE OSSEUSE

(30) Priorität: 20.02.1999 DE 19907354
(43) Veröffentlichungstag der Anmeldung: 14.11.2001
(73) Patentinhaber: Aesculap AG & Co. KG, 78532 Tuttlingen (DE)
(72) Erfinder: VOM BERG, Ingo, D-78647 Trossingen (DE); FINK, Ulrich, D-78532 Tuttlingen (DE); FISCHER, Manfred, D-78532 Tuttlingen (DE); LUTZE, Theodor, D-78582 Balgheim (DE); WEISSHAUPT, Dieter, D-78194 Immendingen (DE)
(74) Vertreter: Böhme, Ulrich, Dr. Dipl.-Phys.
(86) Internationale Anmeldenummer: PCT/EP2000/001261
(87) Internationale Veröffentlichungsnummer: WO 2000/048522

(56) Entgegenhaltungen:
- DE-C- 19 603 887
- DE-C- 19 634 699
- DE-U- 29 903 131
- US-A- 2 576 649

## Beschreibung

Die Erfindung betrifft ein Implantat zur Festlegung einer Knochenplatte in einer Öffnung einer umgebenden Knochenplatte, welches als U-förmige Klammer ausgebildet ist mit einem Steg und zwei quer von diesem abstehenden Anlagearmen, die geeignet sind, die auf eine der Knochenplatten geschobene Klammer derart an dieser Knochenplatte zu halten, daß der Steg vor dem Rand dieser Knochenplatte liegt.

Bei Schädeloperationen werden Zugänge dadurch erzeugt, daß aus dem Schädelknochen plattenförmige Knochenteile herausgesägt werden, die nach Beendigung der Operation in der entstandenen Öffnung der Schädeldecke fixiert werden müssen, damit sie in der alten Position wieder einheilen können. Zur Festlegung dieser Knochenplatten in der Öffnung der Schädeldecke sind Implantate bekannt, die aus mehreren Einzelteilen bestehen, die von der Unterseite und von der Oberseite her an die Schädeldecke und an die eingesetzte Knochenplatte herangeführt werden und die Knochenplatte dadurch in der Schädeldecke fixieren. Obwohl diese bekannten Implantate sehr anwenderfreundlich sind und sich in der Praxis bewährt haben, ist es doch notwendig, mehrteilige Implantate zu verwenden und diese mit Hilfe von relativ komplizierten Applizierwerkzeugen einzusetzen (DE 196 03 887 C2).

In der DE 196 34 699 C1 ist ein Implantat beschrieben, mit dem eine Knochenplatte relativ zu einer anderen Knochenplatte fixiert werden kann. Dieses Implantat weist zwei über einen Steg miteinander verbundene Anlagearme auf, von denen jeder bei eingesetztem Implantat den Spalt zwischen den beiden Knochenplatten überbrückt. Zum Anlegen ist es dabei notwendig, die beiden Anlagearme gegeneinander zu verschwenken, bis sie etwa parallel stehen. Die Arme können auch wieder auseinandergeschwenkt werden, dazu tragen die Arme über den Steg überstehende Verlängerungen. Die Länge des Steges ist bei dem bekannten Implantat wesentlich größer als die Dicke der Knochenplatten, und so stehen die Verlängerungen des eingesetzten Implantates erheblich über die Oberfläche der Knochenplatten hervor. Zum Einsetzen dieses Implantates ist ein Anlegewerkzeug notwendig, da die beiden Anlagearme gegeneinander verschwenkt werden müssen.

In der US-2,576,649 ist weiterhin eine Doppelklammer beschrieben, die durch Verbindung von zwei Einzelklammern hergestellt wird, beispielsweise durch Verschweissen. Diese Klammer ist zum Verbinden von Knochenplatten nur dann geeignet, wenn beide Knochenplatten von der Seite her gegeneinandergeschoben werden können, sie ist aber nicht geeignet, um eine Knochenplatte in eine Öffnung einzusetzen, bei der die einzusetzende Knochenplatte quer zur Ausdehnung der Knochenplatte in die Öffnung eingeführt wird.

Es ist Aufgabe der Erfindung, ein gattungsgemäßes Implantat zu schaffen, das einfacher aufgebaut ist als bekannte Implantate, welches weiterhin ohne spezielle Änlegewerkzeuge verwendet werden kann und welches ein Einsetzen einer Knochenplatte in eine Öffnung einer anderen Knochenplatte ermöglicht, so daß dadurch eine Verschiebung der eingesetzten Knochenplatte in Einsetzrichtung verhindert wird.

Diese Aufgabe wird bei einem Implantat der eingangs beschriebenen Art erfindungsgemäß dadurch gelöst, daß mindestens ein Vorsprung auf der den Anlagearmen abgewandten Außenseite des Stegs zur Festlegung der Klammer an der anderen Knochenplatte so angeordnet ist, daß eine Verschiebung der Klammer relativ zu der anderen Knochenplatte in Steglängsrichtung zumindest in einer Richtung verhindert wird.

Durch die beschriebene Ausgestaltung ist es möglich, auf eine Knochenplatte mehrere derartige als U-förmige Klammer ausgebildete Implantate so aufzuschieben, daß die Klammern an der Knochenplatte gehalten werden und daß der Steg der Klammern vor dem Rand der Knochenplatte liegt. Eine mit beispielsweise drei derartigen Klammern versehene Knochenplatte kann dann in eine Öffnung in der Schädeldecke derart eingesetzt werden, daß die Vorsprünge an den Klammern ein zu tiefes Eintauchen der Knochenplatte in der Öffnung des Schädels verhindern, die Vorsprünge an der Außenseite der Klammer legen somit die Position der Knochenplatte bezüglich der Oberfläche der Schädelöffnung fest, und dies genügt, um die Knochenplatte für den Einheilprozeß zu fixieren. Im übrigen wird die in die Öffnung des Schädels eingelegte Knochenplatte derart in die Schädelöffnung eingelegt, daß eine Markierung an der eingelegten Platte und eine Markierung an der Schädelöffnung zueinander ausgerichtet werden, so daß die Knochenplatte auch in Umfangsrichtung so positioniert wird, wie sie ursprünglich in der Schädelöffnung angeordnet war. Diese Markierungen können beispielsweise durch die beiden Hälften einer Trepanationsbohrung gebildet werden, die zur Vorbereitung des Herausschneidens der Knochenplatte in die Schädeldecke gesetzt wird.

Dabei können die Klammern vorzugsweise an dem die Öffnung in der Schädeldecke verschließenden Knochenteil festgelegt werden, es wäre grundsätzlich aber auch möglich, die Klammern an der Schädeldecke festzulegen und das die Öffnung verschließende Knochenteil dann in die Öffnung einzulegen, durch die Vorsprünge an den an der Schädeldecke gehaltenen Klammern würde ebenfalls ein zu tiefes Eintauchen des Knochenteils verhindert, so daß das Knochenteil dadurch in der Schädelöffnung fixiert ist.

Das neue Implantat wird hier im Zusammenhang mit der Positionierung einer Knochenplatte in einer Schädelöffnung erörtert, es versteht sich aber, daß dieses Implantat immer dann Verwendung finden kann, wenn eine Knochenplatte in einer Öffnung einer anderen Knochenplatte positioniert werden soll.

Wenn die Implantate jeweils nur einen Vorsprung an der Außenseite des Stegs aufweisen, können mit derartigen Implantaten die Knochenplatten normalerweise nur in einer Richtung relativ zueinander fixiert werden, es kann also nur ein zu tiefes Eintauchen einer Knochenplatte in der Öffnung der anderen Knochenplatte verhindert werden, eine beidseitige Fixierung wird dagegen erst möglich, wenn gemäß einer bevorzugten Ausführungsform mehrere Vorsprünge vorgesehen sind, die die andere Knochenplatte gegenüber der Klammer in Steglängsrichtung zentrieren. Durch das Zusammenwirken mehrerer Vorsprünge wird es also möglich, die Verschiebung des Implantats gegenüber der anderen Knochenplatte in beiden Richtungen zu verhindern und dadurch eine eindeutige Positionierung der beiden Knochenplatten zueinander zu erzielen.

Bei einer bevorzugten Ausführungsform ist vorgesehen, daß der Steg an einem Ende an seiner Außenseite einen Vorsprung trägt, dessen Anlagefläche an der anderen Knochenplatte im wesentlichen in einer Ebene mit der Anlagefläche des Anlagearms liegt, der an diesem Ende des Stegs von diesem absteht. Dadurch wird erreicht, daß die beiden relativ zueinander zu fixierenden Knochenplatten in einer Ebene positioniert werden, beispielsweise werden die Außenseiten so zueinander ausgerichtet, daß die in eine Schädelöffnung eingesetzte Knochenplatte genau mit der Schädelaußenseite fluchtet.

Besonders vorteilhaft ist, wenn zumindest ein Teil der Vorsprünge an der Außenseite des Stegs und/oder der Steg selbst elastisch in Richtung der Anlagearme verschiebbar oder verformbar sind. Durch diese elastische Verschiebung oder Verformung kann erreicht werden, daß die Vorsprünge weniger weit vom Rand der Knochenplatte abstehen, an der die Klammer gehalten ist.

Bei Einsetzen der Knochenplatte, an der die Klammern gehalten sind, ergibt sich damit eine Verformung der Stege und/oder der Vorsprünge, so daß nach dem Einsetzen die beiden Knochenplatten durch die elastische Kraft dieser Vorsprünge relativ zueinander fixiert werden. Diese Fixierungskräfte können reine Klemmkräfte sein, die durch die elastische Verformung des Stegs und/oder der Vorsprünge erzeugt werden, es kann sich dabei aber auch um einen Formschluß handeln, dann nämlich, wenn die Vorsprünge beim Einschieben verformt werden und nach Erreichen der Endlage wieder in eine weniger verformte Ausgangsstellung zurückkehren, bei der die Vorsprünge beidseitig an der anderen Knochenplatte anliegen. Selbstverständlich ist auch eine Kombination von Reibschluß und Formschluß möglich.

Bei einer besonders bevorzugten Ausführungsform ist vorgesehen, daß der Steg in Längsrichtung mit einer konkaven Außenseite bogenförmig ausgebildet ist und elastisch in eine gestrecktere Lage verformbar ist. Dadurch legt sich der Steg der Klammer in seinem Mittelbereich an die Knochenplatte an, an der die Klammer gehalten ist, in seinen Endbereichen dagegen an die andere Knochenplatte, und damit erhält man nach der Positionierung der Knochenplatten eine eindeutige Zentrierung und Fixierung der Knochenplatten relativ zueinander.

Günstig ist es, wenn der Steg zumindest an einem Ende über eine gegenüber dem Steg nach außen hin vorspringende Umbiegung in den anschließenden Anlagearm übergeht, die einen Vorsprung zur Festlegung der Klammer an der anderen Knochenplatte ausbildet. Eine solche Umbiegung bildet also selbst einen Vorsprung aus, so daß es nicht notwendig ist, an der Außenseite des Stegs zusätzliche Teile anzuordnen. Eine elastische Verformung auch dieses Vorsprungs ist einmal durch eine bogenförmige Ausgestaltung des Stegs selbst möglich, aber auch durch eine elastische Kompression der Umbiegung.

Es kann vorgesehen sein, daß an einer Seite des Stegs ein Vorsprung durch einen quer vom Steg abstehenden Anlagelappen gebildet wird. In diesem Falle wird an den Steg außenseitig ein Lappen angeformt, beispielsweise durch Anschweißen, der quer von dem Steg absteht und einen Anschlag oder eine Anlagefläche für die andere Knochenplatte ausbildet.

Dabei ist es besonders vorteilhaft, wenn an einer Seite des Stegs ein Vorsprung durch eine Umbiegung und auf der anderen Seite ein weiterer Vorsprung durch einen solchen Anlagelappen gebildet werden.

Der Anlagelappen kann insbesondere Durchbrechungen zur Aufnahme von Knochenschrauben aufweisen, so daß nach dem Einsetzen der Knochenplatten der Anlagelappen über Knochenschrauben an der anderen Knochenplatte festgelegt werden kann, damit erhält man eine zusätzliche und dauerhafte Festlegung der beiden Knochenplatten relativ zueinander.

Derartige Durchbrechungen zur Aufnahme von Knochenschrauben können im übrigen gemäß einer weiteren bevorzugten Ausführungsform auch in den Anlagearmen angeordnet sein, so daß auf diese Weise die Klammern an der Knochenplatte, an der sie durch die Anlagearme gehalten sind, zusätzlich fixiert werden können.

Günstig ist es weiterhin, wenn an der Außenseite des Stegs in dessen mittlerem Bereich über dessen Länge verteilt mehrere Vorsprünge angeordnet sind. Dies Vorsprünge können insbesondere dazu dienen, einen Reibschluß zwischen der Klammer einerseits und der anderen Knochenplatte andererseits herzustellen, außerdem wird dadurch die Zentrierung der beiden Knochenplatten relativ zueinander gefördert.

Insbesondere können diese Vorsprünge im Querschnitt sägezahnförmig ausgebildet sein, so daß die eingesetzte Knochenplatte zwar leicht eingesetzt, nicht aber ohne weiteres wieder herausgezogen werden kann.

Es ist auch möglich, daß die Vorsprünge von einem Ende des Stegs zum anderen zunehmend einen größeren Abstand von der Knochenplatte aufweisen, an der die Anlegearme die Klammer festlegen. Auch dies unterstützt die Zentrierung der Knochenplatten relativ zueinander und erleichtert das Einführen unter Ausbildung von Klemmkräften, die mit der Einschubtiefe zunehmen.

Vorzugsweise sind die Anlagearme federnd auseinanderschwenkbar, so daß die Anlagearme normalerweise federnd an der Oberseite beziehungsweise der Unterseite der Knochenplatte anliegen und die Klammer dadurch zuverlässig an der Knochenplatte festlegen.

Günstig ist es dabei, wenn die Anlagearme an ihrem freien Ende aufeinanderzugerichtete Vorsprünge tragen, so daß dadurch gewisse Unterschiede in der Dicke der Knochenplatten ausgeglichen werden kann.

Insbesondere können die Vorsprünge durch Abbiegungen der Anlagearme gebildet werden.

Die Festlegung der Klammern an der Knochenplatte wird unterstützt, wenn gemäß einer bevorzugten Ausführungsform die Vorsprünge an den Anlagearmen spitz zulaufen.

Die Anlagearme können einteilig ausgebildet sein, bei einer bevorzugten Ausführungsform ist vorgesehen, daß jeder Anlagearm durch vom freien Ende her verlaufende Einschnitte in mindestens zwei nebeneinander angeordnete Einzelarme unterteilt ist. Dadurch werden die Klammern auch gegen eine Verkippung gesichert.

Die Anlagearme können an ihrer der Knochenplatte zugewandten Innenseite eine Profilierung aufweisen, um dort den Kontakt zwischen Anlagearmen und Knochenplatten zu verstärken. Insbesondere kann die Profilierung eine Anzahl von Vorsprüngen mit sägezahnartigem Querschnitt umfassen, so daß das Aufschieben der Klammern erleichtert das Abziehen aber weitgehend verhindert werden.

Das Implantat besteht vorzugsweise aus einem körperverträglichen Metall, beispielsweise aus Titan oder einem Implantatstahl, es kann aber auch vorgesehen sein, daß das Implantat aus einem resorbierbaren Material besteht, so daß nach einer bestimmten Zeit das Implantat abgebaut und vom Körper resorbiert wird.

Die nachfolgende Beschreibung bevorzugter Ausführungsformen der Erfindung dient im Zusammenhang mit der Zeichnung der näheren Erläuterung. Es zeigen:
- Figur 1:: eine Draufsicht auf eine kreisförmige Knochenplatte nach dem Einsetzen in eine kreisförmige Öffnung einer anderen Knochenplatte unter Verwendung von drei unterschiedlich ausgebildeten Klammern;
- Figur 2:: eine Schnittansicht längs Linie 2-2 in Figur 1;
- Figur 3:: eine Ansicht ähnlich Figur 2 bei einem abgewandelten Ausführungsbeispiel einer Klammer;
- Figur 4:: eine Ansicht ähnlich Figur 2 bei einem abgewandelten Ausführungsbeispiel einer Klammer und
- Figur 5:: eine Ansicht ähnlich Figur 2 bei einem abgewandelten Ausführungsbeispiel einer Klammer.

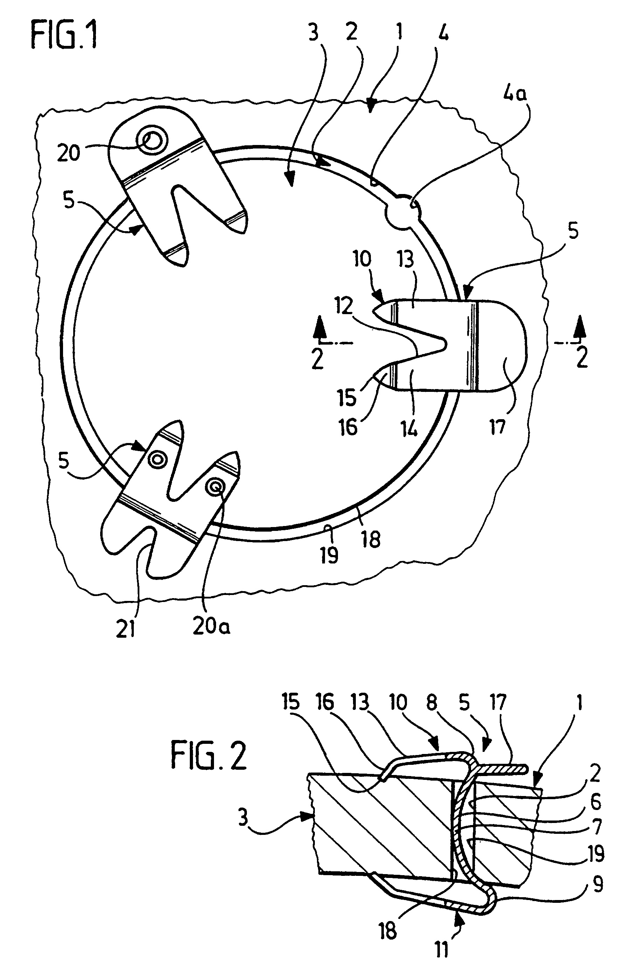

In Figur 1 ist ein Ausschnitt aus dem Schädelknochen 1 dargestellt, aus dem durch einen kreisförmigen Schnitt 2 eine kreisförmige Knochenplatte 3 herausgetrennt ist, die zur Verschließung der dadurch entstandenen Öffnung 4 nach einer erfolgten Operation wieder in diese Öffnung 4 eingesetzt ist. Die kreisförmige Knochenplatte 3 ist dabei derart in die Öffnung 4 eingesetzt, daß die beiden Hälften einer Trepanationsöffnung 4a zueinander ausgerichtet sind. Eine solche Trepanationsöffnung 4a wird vor dem Herausschneiden der kreisförmigen Knochenplatte 3 aus dem Schädelknochen 1 in diesen eingebracht, und von dieser geht die Ausführung des Trennschnitts der Knochenplatte 3 aus.

Zur Fixierung der Knochenplatte 3 in der Öffnung 4 sind auf die Knochenplatte 3 drei Implantate in Form einer U-förmigen Klammer 5 vom Rand her aufgeschoben. Jede Klammer 5 weist einen in Längsrichtung bogenförmig ausgebildeten und damit eine konkave Außenseite 6 aufweisenden Steg 7 auf, der an seinem Ende jeweils über kreisbogenförmige Umbiegungen 8 und 9 in Anlagearme 10 beziehungsweise 11 übergeht (Figur 2). Diese Anlagearme 10 und 11 sind durch V-förmige Einschnitte 12 jeweils in zwei nebeneinander angeordnete Einzelarme 13, 14 unterteilt, diese Einzelarme 13, 14 laufen an ihrem freien Ende in einer Spitze 15 aus, die am Ende einer Abbiegung 16 angeordnet ist, durch die die Einzelarme 13, 14 an ihrem freien Ende in Richtung auf den jeweils anderen Anlegearm umgebogen sind.

In den V-förmigen. Einschnitt 12 kann eine Knochenschraube eingesetzt werden über die die Anlegearme 10 oder 11 gegen die Knochenplatte 3 gepreßt werden, so daß eine optimale Sicherung der Klammer 5 einer Knochenplatte 3 erreichbar ist. Es ist auch möglich, eine solche Knochenschraube durch eine Öffnung 20a hindurchzustecken, die im Anlagearm 10 oder 11 vorgesehen ist, insbesondere in den Einzelarmen 13, 14.

Die Klammer 5 besteht aus einem elastischen Werkstoff, beispielsweise aus Metall wie Titan oder Implantatstahl oder aus einem resorbierbaren Kunststoffmaterial und die Anlagearme 10, 11 können gegen eine elastische Kraft auseinandergeschwenkt werden.

Auf der Außenseite 6 des Stegs 7 ist im Bereich der oberen Umbiegung 8 ein quer vom Steg 7 abstehender Anlagelappen 17 angeformt, der im wesentlichen eben ausgebildet ist und dessen Unterseite im wesentlichen koplanar mit der Unterseite des oberen Anlegearms 10 verläuft.

Die beschriebene Klammer 5 wird benutzt, um die Knochenplatte 3 in der Öffnung 4 des Schädelknochens 1 so zu fixieren, daß der Heilungsprozeß eintreten kann. Zu diesem Zweck werden mehrere derartige Klammern 5, im dargestellten Ausführungsbeispiel 3 derartige Klammern, in Umfangsrichtung versetzt vom Rand her auf die Knochenplatte 3 aufgeschoben, so daß die Anlagearme 10 und 11 an der Oberseite beziehungsweise der Unterseite der Knochenplatte 3 federnd anliegen. Durch die elastische Ausbildung der Klammern 5 können die Anlagearme 10 und 11 entsprechend der Dicke der Knochenplatte unterschiedlich weit aufgebogen werden, in jedem Falle liegen sie danach mit einer Federkraft an der Oberseite beziehungsweise an der Unterseite der Knochenplatte 3 an, wobei die Spitzen 15 der Abbiegungen 16 in Kontakt mit der Knochenplatte 3 gelangen und dadurch die Klammer 5 fest an der Knochenplatte 3 fixieren. Die Fixierung erfolgt dabei so, daß der Steg 7 mit seiner konvexen Innenseite am Rand 18 der Knochenplatte 3 anliegt.

Die in dieser Weise vorbereitete Knochenplatte 3 wird von außen her in die Öffnung 4 des Schädelknochens 1 eingeschoben. Dabei legen sich die unteren Umbiegungen 9 der Stege 7 der drei Klammern 5 an den Rand 19 der Öffnung 4 an, und beim weiteren Eindrücken der Knochenplatte 3 in die Öffnung 4 werden die Umbiegungen 9 in Richtung auf die Knochenplatte 3 verschoben. Dabei ergibt sich eine elastische Verformung des Stegs 7, der dadurch gestreckt wird, und der untere Anlagearm 11 verschiebt sich relativ zur Knochenplatte 3 geringfügig. Dadurch ist es möglich, die Knochenplatte 3 mit den aufgesetzten Klammern 5 in die Öffnung 4 einzuschieben, obwohl die unverformten unteren Umbiegungen 9 geringfügig über die Kontur der Öffnung 4 hervorstehen.

Sobald die Knochenplatte 3 vollständig in die Öffnung 4 eingedrückt ist, kann sich die untere Umbiegung 9 der Klammern 5 an der Unterseite des Schädelknochens 1 wieder radial nach außen bewegen, das heißt die Klammer 5 entspannt sich und der Steg 7 wird wieder stärker abgebogen. Dadurch untergreift die untere Umbiegung 9 den Schädelknochen 1 im Bereich des Rands 19 und zentriert die Klammer 5 relativ zum Schädelknochen 1, die Oberseite des Schädelknochens 1 legt sich an die Unterseite des Anlagelappens 17 an, die Unterseite des Schädelknochens 1 liegt an der Umbiegung 9 an, und die Abmessungen der Klammer 5 und die Anordnung des Anlagelappens 17 und der unteren Umbiegung 9 sind so gewählt, daß in der Endlage der Schädelknochen 1 und die Knochenplatte 3 zueinander ausgerichtet sind (Figur 2).

In der Praxis kann der Chirurg die mit den Klammern 5 versehene Knochenplatte 3 einfach in die Öffnung 4 eindrücken, sobald die Endlage erreicht ist, schnappen die unteren Umbiegungen 9 elastisch in die radial ausgeschobene Stellung, das heißt der Chirurg spürt beim Eindrücken der Knochenplatte 3 das Erreichen der Endposition dadurch, daß sich die Stege 7 und die Umbiegungen 9 plötzlich entspannen, die Knochenplatte 3 rastet also in der Endlage ein. In dieser Endlage ist die Knochenplatte 3 dann auch gegenüber dem Schädelknochen 1 in beiden Richtungen fixiert.

Eine zusätzliche Festlegung läßt sich noch dadurch erreichen, daß die Anlagelappen 17 am Schädelknochen 1 durch Knochenschrauben festgelegt werden. Diese können beispielsweise durch eine Öffnung 20 im Anlagelappen 17 hindurchgreifen oder in einen V-förmigen Einschnitt 21 im Anlagelappen 17 eingesetzt werden (Figur 1). Die drei in Figur 1 dargestellten Klammern 5 unterscheiden sich voneinander durch die Ausgestaltung im Bereich der Anlagelappen 17, ein Anlagelappen 17 weist eine Öffnung 20 auf, ein weiterer einen Einschnitt 21 und bei der dritten Klammer sind keine derartigen Durchbrechungen im Anlagelappen 17 vorgesehen. An einer Knochenplatte können selbstverständlich wahlweise gleichartige oder verschiedene Klammern festgelegt werden.

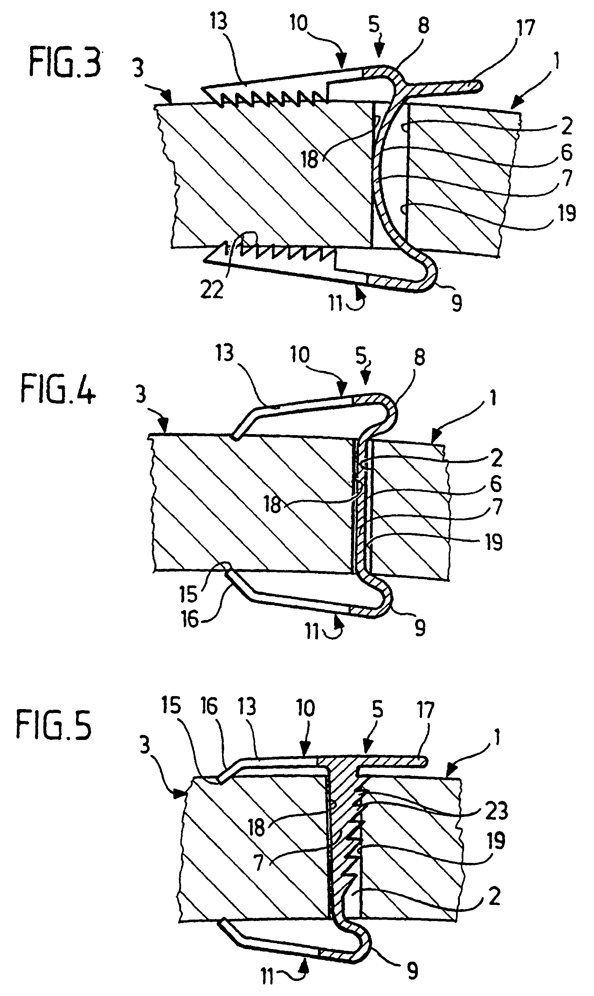

Das Ausführungsbeispiel der Figur 3 entspricht dem der Figur 2, gleiche Teile tragen daher die gleichen Bezugszeichen.

Im Unterschied zum Ausführungsbeispiel der Figur 2 fehlen bei den Anlagearmen 10, 11 Spitzen 15 und Abbiegungen 16, statt dessen sind die Anlagearme 10 und 11 an ihrer der Knochenplatte 3 zugewandten Innenseite mit sägezahnartigen Vorsprüngen 22 versehen, die ein leichtes Aufschieben der Anlagearme 10, 11 ermöglichen, die sich aber dem Abziehen der Klammer 5 widersetzen.

Das Ausführungsbeispiel der Figur 4 ist wiederum ähnlich aufgebaut wie das der Figur 2, einander entsprechende Teile tragen ebenfalls dieselben Bezugszeichen.

Im Unterschied zum Ausführungsbeispiel der Figur 2 fehlt bei der Klammer der Figur 4 der Anlagelappen 17. Dessen Anschlagfunktion wird übernommen von der oberen Umbiegung 8, die beiden Umbiegungen 8 und 9 bilden bei dieser Klammer Vorsprünge, zwischen denen der Schädelknochen 1 relativ zur Klammer 5 zentriert wird. Um dies zu erreichen, kann vorgesehen sein, daß der Steg 7 im Bereich zwischen den Umbiegungen 8, 9 geradlinig ausgebildet ist, unbedingt notwendig ist dies allerdings nicht, der Steg könnte in ähnlicher Weise wie im Ausführungsbeispiel 2 dargestellt, gebogen ausgeführt werden.

Auch beim Ausführungsbeispiel der Figur 5 ist ein ähnlicher Aufbau der Klammer 5 vorgesehen, einander entsprechende Teile tragen daher wieder dieselben Bezugszeichen wie beim Ausführungsbeispiel der Figur 2.

Bei diesem Ausführungsbeispiel fehlt die obere Umbiegung 8, der obere Anlagearm 10 geht bei diesem Ausführungsbeispiel unmittelbar in den Anlagelappen 17 über. Der Steg 7 ist geradlinig ausgebildet und trägt an seiner der Knochenplatte 3 abgewandten Außenseite eine Anzahl von im Querschnitt sägezahnartigen Vorsprüngen 23, die Dicke des Stegs 7 nimmt von der unteren Umbiegung 9 zum Anlagelappen 17 hin zu.

Beim Einsetzen der Knochenplatte 3 mit einer Klammer gemäß Figur 5 ergibt sich eine Zentrierung des Schädelknochens 1 zwischen der unteren Umbiegung 9 und dem Anlagelappen 17, außerdem legen sich die Vorsprünge 23 zunehmend kräftig an den Schädelknochen 1 an und vermitteln somit einen Reibschluß oder eine Klemmverbindung zwischen Knochenplatte 3 und Schädelknochen 1. Damit ist eine Fixierung durch kombinierten Reib- und Formschluß erreicht.

Grundsätzlich wäre es auch möglich, den Steg 7 beim Ausführungsbeispiel der Figur 5 unmittelbar in den unteren Anlagearm 11 übergehenzulassen, man könnte also darauf verzichten, daß die untere Umbiegung 9 einen Zentriervorsprung bildet. In diesem Falle würden Knochenplatte 3 und Klammern 5 in der Öffnung 4 im wesentlichen durch einen Klemmschluß gehalten, wobei die Eintauchtiefe durch den Anlagelappen 17 begrenzt wird.

## Patentansprüche

1. Implantat zur Festlegung einer Knochenplatte (3) in einer Öffnung einer umgebenden Knochenplatte (1), welches als U-förmige Klammer (5) ausgebildet ist mit einem Steg (7) und zwei quer von diesem abstehenden Anlagearmen (10, 11), die geeignet sind, die auf eine der Knochenplatten (3) geschobene Klammer (5) derart an dieser Knochenplatte (3) zu halten, daß der Steg (7) vor dem Rand dieser Knochenplatte (3) liegt, **dadurch gekennzeichnet, daß** mindestens ein Vorsprung (8, 9; 17; 23) auf der den Anlagearmen (10, 11) abgewandten Außenseite (6) des Stegs (7) zur Festlegung der Klammer (5) an der anderen Knochenplatte (1) so angeordnet ist, daß eine Verschiebung der Klammer (5) relativ zu der anderen Knochenplatte (1) in Steglängsrichtung zumindest in einer Richtung verhindert wird.

2. Implantat nach Anspruch 1, **dadurch gekennzeichnet, daß** mehrere Vorsprünge (8, 9; 17) vorgesehen sind, die die andere Knochenplatte (1) gegenüber der Klammer (5) in Steglängsrichtung zentrieren.

3. Implantat nach einem der voranstehenden Ansprüche, **dadurch gekennzeichnet, daß** der Steg (7) an einem Ende an seiner Außenseite (6) einen Vorsprung (8; 17) trägt, dessen Anlagefläche an der anderen Knochenplatte (1) im wesentlichen in einer Ebene mit der Anlagefläche des Anlagearms (10) liegt, der an diesem Ende des Stegs (7) von diesem absteht.

4. Implantat nach einem der voranstehenden Ansprüche, **dadurch gekennzeichnet, daß** zumindest ein Teil der Vorsprünge (8, 9; 17; 23) an der Außenseite (6) des Stegs (7) und/oder der Steg (7) selbst elastisch in Richtung der Anlagearme (10, 11) verschiebbar oder verformbar sind.

5. Implantat nach Anspruch 4, **dadurch gekennzeichnet, daß** der Steg (7) in Längsrichtung mit einer konkaven Außenseite (6) bogenförmig ausgebildet ist und elastisch in eine gestrecktere Lage verformbar ist.

6. Implantat nach einem der voranstehenden Ansprüche, **dadurch gekennzeichnet, daß** der Steg (7) zumindest an einem Ende über eine gegenüber dem Steg (7) nach außen hin vorspringende Umbiegung (8, 9) in den anschließenden Anlagearm (10 beziehungsweise 11) übergeht, die einen Vorsprung zur Festlegung der Klammer (5) an der anderen Knochenplatte (1) ausbildet.

7. Implantat nach Anspruch 6, **dadurch gekennzeichnet, daß** an einer Seite des Stegs (7) ein Vorsprung durch eine Umbiegung (9) und auf der anderen Seite ein weiterer Vorsprung durch einen quer vom Steg (7) abstehenden Anlagelappen (17) gebildet werden.

8. Implantat nach Anspruch 7, **dadurch gekennzeichnet, daß** der Anlagelappen (17) Durchbrechungen (20, 21) zur Aufnahme von Knochenschrauben aufweist.

9. Implantat nach einem der voranstehenden Ansprüche, **dadurch gekennzeichnet, daß** an der Außenseite (6) des Stegs (7) in dessen mittlerem Bereich über dessen Länge verteilt mehrere Vorsprünge (23) angeordnet sind.

10. Implantat nach Anspruch 9, **dadurch gekennzeichnet, daß** die Vorsprünge (23) im Querschnitt sägezahnartig ausgebildet sind.

11. Implantat nach Anspruch 9 oder 10, **dadurch gekennzeichnet, daß** die Vorsprünge (23) von einem Ende des Stegs (7) zum anderen zunehmend einen größeren Abstand von der Knochenplatte (3) aufweisen, an der die Anlagearme (10, 11) die Klammer (5) festlegen.

12. Implantat nach einem der voranstehenden Ansprüche, **dadurch gekennzeichnet, daß** die Anlagearme (10, 11) federnd auseinanderschwenkbar sind.

13. Implantat nach einem der voranstehenden Ansprüche, **dadurch gekennzeichnet, daß** die Anlagearme (10, 11) an ihrem freien Ende aufeinanderzugerichtete Vorsprünge (16) tragen.

14. Implantat nach Anspruch 13, **dadurch gekennzeichnet, daß** die Vorsprünge durch Abbiegungen (16) der Anlagearme (10, 11) gebildet werden.

15. Implantat nach einem der Ansprüche 13 oder 14, **dadurch gekennzeichnet, daß** die Vorsprünge (14) an den Anlagearmen (10, 11) spitz zulaufen.

16. Implantat nach einem der voranstehenden Ansprüche, **dadurch gekennzeichnet, daß** jeder Anlagearm (10, 11) durch vom freien Ende her verlaufende Einschnitte (12) in mindestens zwei nebeneinander angeordnete Einzelarme (13, 14) unterteilt ist.

17. Implantat nach einem der voranstehenden Ansprüche, **dadurch gekennzeichnet, daß** in einem oder beiden Anlagearmen (10, 11) Durchbrechungen (12; 20a) zur Aufnahme von Knochenschrauben angeordnet sind.

18. Implantat nach einem der voranstehenden Ansprüche, **dadurch gekennzeichnet, daß** die Anlagearme (10, 11) an ihrer der Knochenplatte (3) zugewandten Innenseite eine Profilierung (22) aufweisen.

19. Implantat nach Anspruch 18, **dadurch gekennzeichnet, daß** die Profilierung eine Anzahl von Vorsprüngen (22) mit sägezahnartigem Querschnitt umfaßt.

20. Implantat nach einem der voranstehenden Ansprüche, **dadurch gekennzeichnet, daß** es aus einem körperverträglichen Metall besteht.

21. Implantat nach einem der Ansprüche 1 bis 19, **dadurch gekennzeichnet, daß** es aus einem resorbierbaren Material besteht.

## Claims

1. An implant for fixing a bone flap (3) within an opening in a surrounding sheet of bone (1), being designed as a U-shaped clip (5) with a bridge member (7) and two contacting arms (10, 11) which project transversely therefrom and which, once the clip (5) has been pushed on to one of the bone flaps (3), are suitable for holding it on the said bone flap (3) in such a way that the bridge member (7) lies in front of the margin of this bone flap (3), **characterised in that**, in order to attach the clip (5) to the sheet of bone (1), at least one projection (8, 9; 17; 23) on the outer surface (6) of the bridge member (7), which outer surface (6) faces away from the contacting arms (10, 11), is arranged in such a way that displacement of the clip (5) relative to the sheet of bone (1) in the longitudinal direction of the bridge member is prevented in at least one direction.

2. An implant according to Claim 1, **characterised in that** a plurality of projections (8,9; 17) which centre the sheet of bone (1) relative to the clip (5) in the longitudinal direction of the bridge member are provided.

3. An implant according to one of the preceding claims, **characterised in that** the bridge member (7) bears, at one end on its outer surface (6), a projection (8; 17) the surface of which in contact with the sheet of bone (1) is substantially within the same plane as the contacting surface of the contacting arm (10) which projects from the bridge member (7) at this end of thereof.

4. An implant according to one of the preceding claims, **characterised in that** at least some of the projections (8,9; 17; 23) on the outer surface (6) of the bridge member (7) and/or the bridge member (7) itself are elastically displaceable or deformable in the direction of the contacting arms (10, 11).

5. An implant according to Claim 4, **characterised in that**, in the longitudinal direction, the bridge member (7) is of an arcuate design with a concave outer surface (6) and is deformable into a flatter state.

6. An implant according to one of the preceding claims, **characterised in that**, at least at one end, the bridge member (7) undergoes a transition into the adjoining contacting arm (10 or 11) via a bend (8, 9) which projects outwards relative to the bridge member (7) and which forms a projection for attaching the clip (5) to the other sheet of bone (1).

7. An implant according to Claim 6, **characterised in that** a projection on one side of the bridge member (7) is formed by a bend (9) and another projection on the other side is formed by a contacting tongue (17) projecting transversely from the bridge member (7).

8. An implant according to Claim 7, **characterised in that** the contacting tongue (17) has perforations (20, 21) for accommodating bone screws.

9. An implant according to one of the preceding claims, **characterised in that** a plurality of projections (23) are arranged on the outer surface (6) of the bridge member (7) distributed along the length thereof in the central region thereof.

10. An implant according to Claim 9, **characterised in that** the projections (23) are in the form of saw teeth in cross-section.

11. An implant according to Claim 9 or 10, **characterised in that**, from one end of the bridge member (7) to the other, the projections (23) are positioned at an increasingly greater distance from the bone flap (3) to which the clip (5) is attached by the contacting arms (10, 11).

12. An implant according to one of the preceding claims, **characterised in that** the contacting arms (10, 11) maybe elastically swivelled apart.

13. An implant according to one of the preceding claims, **characterised in that** the contacting arms (10, 11) bear projections (16) on their free end which point towards one another.

14. An implant according to Claim 13, **characterised in that** the projections are formed by kinks (16) in the contacting arms (10, 11).

15. An implant according to one of Claims 13 or 14, **characterised in that** the projections (14) on the contacting arms (10, 11) narrow to a point.

16. An implant according to one of the preceding claims, **characterised in that** each contacting arm (10, 11) is subdivided, by notches (12) extending from the free end, into at least two individual arms (13, 14) arranged next to each other.

17. An implant according to one of the preceding claims, **characterised in that** perforations (12; 20a) are arranged in one or both contacting arms (10, 11) for the purpose of accommodating bone screws.

18. An implant according to one of the preceding claims, **characterised in that** the contacting arms (10, 11) have a shaped contour (22) on their inner surface which faces the bone flap (3).

19. An implant according to Claim 18, **characterised in that** the shaped contour includes a number of projections (22) of saw-tooth-shaped cross-section.

20. An implant according to one of the preceding claims, **characterised in that** it is composed of a physiologically compatible metal.

21. An implant according to one of Claims 1 to 19, **characterised in that** it is composed of an absorbable material.

## Revendications

1. Implant pour fixer une plaque osseuse (3) dans une ouverture d'une plaque osseuse (1) environnante, qui est réalisé sous forme d'agrafe (5) en forme de U avec une traverse (7) et deux bras d'appui (10, 11) faisant saillie transversalement hors de celle-ci, qui sont appropriés à maintenir sur une des plaques osseuses (3) l'agrafe (5) enfilée sur cette plaque osseuse (3) de telle sorte que la traverse (7) se trouve devant le bord de cette plaque osseuse (3), **caractérisé en ce qu'**au moins une saillie (8, 9 ; 17 ; 23) est agencée sur la face extérieure (6), détournée des bras d'appui (10, 11), de la traverse (7) pour fixer l'agrafe (5) sur l'autre plaque osseuse (1) de telle sorte qu'un déplacement de l'agrafe (5) par rapport à l'autre plaque osseuse (1) est empêché en direction longitudinale de la traverse, au moins dans une direction.

2. Implant selon la revendication 1, **caractérisé en ce qu'**il est prévu plusieurs saillies (8, 9 ; 17) qui centrent l'autre plaque osseuse (1) par rapport à l'agrafe (5) en direction longitudinale de la traverse.

3. Implant selon l'une des revendications précédentes, **caractérisé en ce que** la traverse (7) porte à une extrémité sur sa face extérieure (6) une saillie (8 ; 17) dont la surface d'appui sur l'autre plaque osseuse (1) se trouve sensiblement dans un plan avec la surface d'appui du bras d'appui (10) qui fait saillie à cette extrémité de la traverse (7) hors de cette dernière.

4. Implant selon l'une des revendications précédentes, **caractérisé en ce qu'**au moins une partie des saillies (8, 9 ; 17 ; 23) sur la face extérieure (6) de la traverse (7) et/ou la traverse (7) elle-même sont déplaçables ou déformables élastiquement en direction des bras d'appui (10, 11).

5. Implant selon la revendication 4, **caractérisé en ce que** la traverse (7) est réalisée en direction longitudinale en forme d'arc avec une face extérieure (6) concave et peut être déformée élastiquement jusque dans une position plus étirée.

6. Implant selon l'une des revendications précédentes, **caractérisé en ce qu'**au moins à une extrémité, la traverse (7) se transforme, via un coudage (8, 9) en saillie vers l'extérieur par rapport à la traverse (7), dans le bras d'appui adjacent (10 respectivement 11), coudage qui forme une saillie pour fixer l'agrafe (5) sur l'autre plaque osseuse (1).

7. Implant selon la revendication 6, **caractérisé en ce que** sur un côté de la traverse (7), une saillie est formée par un coudage (9) et sur l'autre côté, une autre saillie est formée par une patte d'appui (17) faisant saillie transversalement à la traverse (7).

8. Implant selon la revendication 7, **caractérisé en ce que** la patte d'appui (17) présente des percées (20, 21) pour recevoir des vis à os.

9. Implant selon l'une des revendications précédentes, **caractérisé en ce que** sur la face extérieure (6) de la traverse (7), plusieurs sailles (23) sont agencées dans sa région médiane et réparties sur sa longueur.

10. Implant selon la revendication 9, **caractérisé en ce que** les saillies (23) sont réalisées en dents de scie en section transversale.

11. Implant selon l'une ou l'autre des revendications 9 et 10, **caractérisé en ce que** les saillies (23) présentent, depuis une extrémité de la traverse (7) à l'autre, une distance de plus en plus grande vis-à-vis de la plaque osseuse (3) sur laquelle les bras d'appui (10, 11) fixent l'agrafe (5).

12. Implant selon l'une des revendications précédentes, **caractérisé en ce que** les bras d'appui (10, 11) peuvent être pivotés élastiquement en éloignement l'un de l'autre.

13. Implant selon l'une des revendications précédentes, **caractérisé en ce que** les bras d'appui (10, 11) portent à leur extrémité libre des saillies (16) orientées l'une vers l'autre.

14. Implant selon la revendication 13, **caractérisé en ce que** les saillies sont formées par des pliures (16) des bras d'appui (10, 11).

15. Implant selon l'une ou l'autre des revendications 13 et 14, **caractérisé en ce que** les saillies (14) convergent en pointe sur les bras d'appui (10, 11).

16. Implant selon l'une des revendications précédentes, **caractérisé en ce que** chaque bras d'appui (10, 11) est subdivisé, par des encoches (12) s'étendant depuis l'extrémité libre, en au moins deux bras (13, 14) individuels agencés l'un à côté de l'autre.

17. Implant selon l'une des revendications précédentes, **caractérisé en ce que** dans un ou dans les deux bras d'appui (10, 11) sont agencées des percées (12 ; 20a) pour recevoir des vis à os.

18. Implant selon l'une des revendications précédentes, **caractérisé en ce que** les bras d'appui (10, 11) présentent un profilage (22) sur leur face intérieure tournée vers la plaque osseuse (3).

19. Implant selon la revendication 18, **caractérisé en ce que** le profilage comprend un certain nombre de saillies (22) avec section transversale en forme de dents de scie.

20. Implant selon l'une des revendications précédentes, **caractérisé en ce que** qu'il est constitué par un métal toléré par le corps.

21. Implant selon l'une des revendications 1 à 19, **caractérisé en ce qu'**il est constitué par un matériau résorbable.
